# EUROPEAN PATENT APPLICATION

(11) **EP 3 124 593 A1**
(43) Date of publication of application: **01.02.2017**
(21) Application number: 14886801.1
(22) Date of filing: 17.07.2014
(51) Int. Cl.: C12M 1/26, C12M 1/00

(54) **CELL CAPTURING APPARATUS, CELL CAPTURING DEVICE PROVIDED WITH PRE-PROCESSING PART, AND PRE-PROCESSING PART**

(30) Priority: 28.03.2014 JP 2014068168
(71) Applicant: Hitachi Chemical Co., Ltd., Chiyoda-ku Tokyo 100-6606 (JP)
(72) Inventor: KIKUHARA Yoshihito, Tokyo 100-6606 (JP); ODAGIRI Taihei, Tokyo 100-6606 (JP); KOTATO Akio, Tokyo 100-6606 (JP); KANETOMO Masafumi, Tokyo 168-0082 (JP)
(74) Representative: Hoffmann Eitle
(86) International application number: PCT/JP2014/069067
(87) International publication number: WO 2015/145793

(57) **Abstract**

By providing a pre-processing unit 20A on a processing liquid channel included in a cell capturing apparatus 100, air bubbles accumulate in an introduction region 24 inside of the pre-processing unit 20A and thus the air bubbles are removed while a processing liquid moves through the introduction region 24 and a discharge region 26, even when the air bubbles are contained in the processing liquid. By providing a foreign matter removal filter 23, at least a part of foreign matters (foreign matters that cannot pass through through-holes 21) contained in the processing liquid can be captured and thus the processing liquid from which the foreign matters are removed can be supplied to a cell capturing device 1 at the subsequent stage. Consequently, the supply of the processing liquid with air bubbles mixed toward the vicinity of a filter 57 of the cell capturing device 1 disposed at the stage subsequent to the pre-processing unit 20A is reduced. This allows the process of cell capturing to be carried out with good repeatability.

## Description

### Technical Field

The present invention relates to a cell capturing apparatus, a cell capturing device provided with a pre-processing part, and a pre-processing part for capturing specific cells penetrating into a test liquid with a filter.

### Background Art

Cancer, which is also called "malignant tumor", causes serious trouble for the life support of a living body when the cancer is progressed and thus various treatment methods of cancer have been studied. The presence or absence of metastasis of cancer acts as a guide for determining the treatment policy of cancer patients. The metastasis is caused by cancer cells penetrating into blood vessels or lymph vessels, spreading throughout the body through the blood vessels or the lymph vessels, and migrating to other organs. Consequently, the measurement of the presence or absence of cancer cells and the amount of the cancer cells in the blood is important information at the time of the metastasis prediction of cancer. The cancer cell circulating in a human body through blood vessels or lymph vessels as described above is called Circulating Tumor Cell (CTC).

As conventional arts for capturing CTCs for the inspection of metastasis cancer or the evaluation of anticancerous, for example, a method for capturing CTCs with a filter has been known as described in Patent Literature 1. Patent Literature 1 has described a production method using semiconductor technology of the filter, the shape of a cell unit housing the filter, and a structure of channels through which blood and a processing liquid are flowed. Specifically, a configuration in which cancer cells are captured by flowing the blood containing the cancer cells through the cell unit housing the filter and identifying the captured cancer cells existing on the filter as cancer cells by staining is described. Patent Literatures 2 and 3 have disclosed a body fluid processing system including a cell processing cartridge that can provide at least one of the physical action, chemical action, and physiological action to either cancer cells or immune cells by passing a cell dispersion liquid containing either the cancer cells or the immune cells. Patent Literature 4 has disclosed a microfluidic devices provided with a polydimethylsiloxane (PDMS) upper member provided with a sample supply port provided at the upper part and the lower part of a nickel substrate having fine through-holes in the nickel substrate and a lower member provided with a sample discharge port.

### Citation List

### Patent Literature

Patent Literature 1: U.S. Patent Application Publication No. 2011/0053152
Patent Literature 2: Japanese Unexamined Patent Publication No. 2010-227011
Patent Literature 3: Japanese Unexamined Patent Publication No. 2010-75191
Patent Literature 4: Japanese Unexamined Patent Publication No. 2011-163830

### Summary of Invention

### Technical Problem

When the apparatuses described in Patent Literatures 1 to 4 are used, however, the processing efficiency of a test body may be significantly deteriorated and measurement accuracy may be reduced when air is mixed in the flow channel of the apparatus. In addition, the cell capture may be disturbed due to accumulation of foreign matters in the processing liquid on the filter.

The present invention has been made in view of the above problems and a purpose of the present invention is to provide a cell capturing apparatus, a cell capturing device provided with a pre-processing unit, and a pre-processing unit in which mixing of air in the vicinity of a filter for capturing cells is particularly reduced and foreign matters in the liquid are removed and thus the process relating to capturing cells can be carried out with good repeatability.

### Solution to Problem

In order to solve the problem, the inventors of the present invention have made intensive study, and as a result, found that many air bubbles are generated immediately after the processing liquid and the test liquid move from an introduction channel to an introduction region of the cell capturing device. The inventors have considered that the inner pressures of the processing liquid and the test liquid are temporally decreased when the liquid emerge from the narrow introduction channel to the wider introduction region, and therefore, the air bubbles dissolved in the liquid are generated by being separated therefrom, whereby the inventors of the present invention have accomplished the present invention.

Specifically, a cell capturing apparatus according to one embodiment of the present invention includes: a test liquid storage container configured to store a test liquid containing cells; a cell capturing device including an introduction channel configured to introduce the test liquid or a processing liquid for processing the cells in the test liquid into an inside, a discharge channel configured to discharge the test liquid or the processing liquid to an outside, and a filter provided with a plurality of through-holes in a thickness direction, the filter being disposed on a channel between the introduction channel and the discharge channel so that the test liquid or the processing liquid passes through the through-holes; a test liquid channel configured to connect the test liquid storage container and the introduction channel of the cell capturing device; a processing liquid storage container configured to store the processing liquid for processing cells captured on the filter by passing through the filter; a processing liquid channel configured to connect the processing liquid storage container and the introduction channel of the cell capturing device; means for selection configured to select a liquid supplied to the cell capturing device from the test liquid and the processing liquid; means for liquid supply configured to supply the test liquid from the test liquid storage container or the processing liquid from the processing liquid storage container to the cell capturing apparatus based on a selection result of the means for selection; and a pre-processing unit including an air bubble accumulated region formed therein and disposed on the processing liquid channel and/or on the test liquid channel, the air bubble accumulated region having a larger diameter than a diameter of the provided channel, and including a foreign matter removal filter provided with a plurality of through-holes in a thickness direction, the foreign matter removal filter being disposed in the air bubble accumulated region so that the processing liquid or the test liquid passes through the through-holes.

As the cell capturing apparatus, the inner pressures of the processing liquid and the test liquid are temporarily decreased by providing, on the processing liquid channel or the test liquid channel, a pre-processing unit provided with the air bubble accumulated region having a larger diameter than a diameter of the channels inside of the pre-treatment unit. As a result, the air bubbles mixed in the processing liquid flowed through the processing liquid channel or the test liquid flowed through the test liquid channel are separated and accumulated in the air bubble accumulated region. Here, the specific gravity of the air bubbles is smaller than that of the liquids and thus the separated air bubbles move to the upper part of the liquids to accumulate in the air bubble accumulated region. Consequently, the supply of the processing liquid with air bubbles mixed toward the vicinity of the filter of the cell capturing device disposed at the stage subsequent to the pre-processing unit is reduced. This allows the process of cell capturing to be carried out with good repeatability. The pre-processing unit is provided with the foreign matter removal filter. This allows the air bubbles to be removed with high accuracy by accumulating the air bubbles that cannot rapidly move to the upper part due to being involved in the flow of the liquid but can move to the discharge port. If foreign matters are mixed in the processing liquid, the foreign matters can be removed and thus processing efficiency of capturing cells on the filter of the cell capturing device can be improved.

Here, when an introduction port of the processing liquid or of the test liquid to the air bubble accumulated region is disposed on an upper side above a discharge port of the air bubble accumulated region in a height direction, the processing liquid or the test liquid having high specific gravity flows to the discharge port disposed at the lower part. This is preferable because the separation of the liquid and the air bubbles having low specific gravity is promoted.

An aspect in which a main component of the foreign matter removal filter is a metal is preferable. When the main component of the foreign matter removal filter is a metal, the thickness of the filter can be thinner by taking advantage of its high rigidity.

When the pre-processing unit is provided on the processing liquid channel, a cross-sectional area viewed in a moving direction of the processing liquid in the air bubble accumulated region is preferably equal to or larger than a cross-sectional area viewed in a moving direction of the processing liquid in the introduction channel. When the pre-processing unit is provided on the test liquid channel, a cross-sectional area viewed in a moving direction of the test liquid in the air bubble accumulated region is preferably equal to or larger than the cross-sectional area viewed in a moving direction of the test liquid in the introduction channel.

By employing the structure as described above, the inner pressure of the processing liquid or of the test liquid that has reached the air bubble accumulated region is dropped at once and thus the air bubbles dissolved in the liquid are separated and the separated air bubbles tend to accumulate. This allows the effect of air bubble removal from the processing liquid to be improved. By providing the pre-processing unit as described above on the upstream side of the introduction channel of the filter of the cell capturing device, air bubbles can be previously removed before the processing liquid or the test liquid passes through the filter of the cell capturing device. To the cross-sectional area of the processing liquid channel or of the test liquid channel, the cross-sectional area of the air bubble accumulated region is equal to or larger than the cross-sectional area viewed in the moving direction of the processing liquid or of the test liquid in the cell capturing device. This is preferable because the air bubbles that would be generated in the cell capturing device in the case of a conventional structure that does not provide the pre-processing unit can be completely captured in the air bubble accumulated region in the pre-processing unit.

An aspect in which the pre-processing unit is configured by a member having the same shape as the shape of the cell capturing device except the filter is preferable.

As described above, when the aspect in which the pre-processing unit is constituted by the member having the same shape as the shape of the cell capturing device is employed, for example, the production cost of the pre-processing unit can be reduced compared with the case of producing a pre-processing unit having a shape different from the shape of the cell capturing device.

The pre-processing unit is preferably provided on each of the processing liquid channel and the test liquid channel. By providing the pre-processing unit on each of the processing liquid channel and the test liquid channel, the pre-processing unit on the processing liquid channel can be replaced for each test liquid, and the pre-processing unit on the test liquid channel can be repeatedly used.

The function of the pre-processing unit included in the cell capturing apparatus according to one embodiment of the present invention can be involved in the cell capturing device to form a cell capturing device provided with a pre-processing unit. Such a cell capturing device provided with a pre-processing unit is substantially the same invention as the cell capturing apparatus and has a similar action and effect.

More specifically, a cell capturing device provided with a pre-processing unit according to one embodiment of the present invention includes: one or more introduction channels configured to introduce a test liquid containing cells or a processing liquid for processing the cells in the test liquid into an inside; a discharge channel configured to discharge the test liquid or the processing liquid to an outside; a filter provided with a plurality of through-holes in a thickness direction and disposed on a channel between the introduction channel and the discharge channel so that the test liquid or the processing liquid passes through the through-holes; and a pre-processing unit including an air bubble accumulated region formed therein and disposed on at least one introduction channel, the air bubble accumulated region having a larger diameter than a diameter of the introduction channel, and including a foreign matter removal filter provided with a plurality of through-holes in a thickness direction, the foreign matter removal filter being disposed in the air bubble accumulated region so that the processing liquid or the test liquid passes through the through-holes.

By providing the cell capturing device provided with a pre-processing unit with the pre-processing unit that can remove air bubbles, a more compact structure can be achieved.

Here, an introduction port of the test liquid or of the processing liquid to the air bubble accumulated region in the pre-processing unit is preferably disposed on an upper side above a discharge port of the air bubble accumulated region in a height direction.

A main component of the foreign matter removal filter is preferably a metal.

When the pre-processing unit is provided on a processing liquid channel through which the processing liquid flows in the introduction channels, a cross-sectional area viewed in a moving direction of the processing liquid in the air bubble accumulated region is preferably equal to or larger than a cross-sectional area viewed in a moving direction of the processing liquid in the introduction channel. When the pre-processing unit is provided on a test liquid channel through which the test liquid flows in the introduction channels, a cross-sectional area viewed in a moving direction of the test liquid in the air bubble accumulated region is preferably equal to or larger than a cross-sectional area viewed in a moving direction of the test liquid in the introduction channel.

The plurality of introduction channels are preferably provided, and the pre-processing unit is preferably provided on each introduction channel.

The present invention can also be an invention relating to the pre-processing unit.

More specifically, a pre-processing unit according to one embodiment of the present invention includes: an introduction channel configured to introduce a test liquid containing cells into an inside; an air bubble accumulated region formed therein and disposed on the introduction channel, the air bubble accumulated region having a larger diameter than a diameter of the introduction channel; and a foreign matter removal filter provided with a plurality of through-holes in a thickness direction, the foreign matter removal filter being disposed in the air bubble accumulated region so that the test liquid passes through the through-holes, in which a hole diameter of the foreign matter removal filter is 15 µm to 100 µm.

An aspect in which, in the foreign matter removal filter, an introduction port of the test liquid to the air bubble accumulated region is disposed on an upper side above a discharge port of the air bubble accumulated region in a height direction is preferable.

A pre-processing unit according to one embodiment of the present invention includes: one or more introduction channels configured to introduce a processing liquid for processing cells in a test liquid into an inside; an air bubble accumulated region formed therein and disposed on each introduction channel, the air bubble accumulated region having a larger diameter than a diameter of the introduction channel; and a foreign matter removal filter provided with a plurality of through-holes in a thickness direction, the foreign matter removal filter being disposed in the air bubble accumulated region so that the test liquid passes through the through-holes, in which a hole diameter of the foreign matter removal filter is 1 µm to 5 µm.

An aspect in which, in the foreign matter removal filter, the introduction port of the processing liquid to the air bubble accumulated region is disposed on an upper side above a discharge port of the air bubble accumulated region in a height direction is preferable.

An aspect in which a main component of the foreign matter removal filter is a metal is preferable.

An aspect in which a cross-sectional area viewed in a moving direction of the test liquid in the air bubble accumulated region is equal to or larger than a cross-sectional area viewed in a moving direction of the test liquid in the introduction channel is preferable.

An aspect in which a cross-sectional area viewed in a moving direction of the processing liquid in the air bubble accumulated region is equal to or larger than a cross-sectional area viewed in a moving direction of the processing liquid in the introduction channel is preferable.

### Advantageous Effects of Invention

According to the present invention, a cell capturing apparatus, a cell capturing device provided with a pre-processing unit, and a pre-processing unit are provided in which mixing of air in the vicinity of a filter for capturing cells is particularly reduced and thus the process relating to capturing cells can be carried out with good repeatability.

### Brief Description of Drawings

FIG. 1 is a view illustrating a configuration of a cell capturing apparatus according to an embodiment of the present invention.
FIG. 2 is a view illustrating a configuration of a cell capturing device included in the cell capturing apparatus.
FIG. 3 is a view illustrating a configuration of a pre-processing unit included in the cell capturing apparatus.
FIG. 4 is a view illustrating a configuration of a cell capturing device provided with a pre-processing unit according to a modified example.
FIG. 5 is a view illustrating a configuration of a pre-processing unit according to the modified example.
FIG. 6 is a view illustrating a configuration of a pre-processing unit according to the modified example.
FIG. 7 is a view illustrating a configuration of a pre-processing unit according to the modified example.
FIG. 8 is a view illustrating a configuration of a pre-processing unit according to the modified example.
FIG. 9 is a view illustrating a configuration of a pre-processing unit according to the modified example.
FIG. 10 is a view illustrating a configuration of a pre-processing unit according to the modified example.

### Description of Embodiments

Hereinafter, an embodiment for carrying out the present invention will be described in detail with reference to the attached drawings. In the description of the drawings, the same sign is assigned to the same element and redundant descriptions are omitted. In this specification, numerical ranges indicated with "to" represent ranges including the minimum values and maximum values that are values described before and after "to", respectively.

### Cell Capturing Apparatus

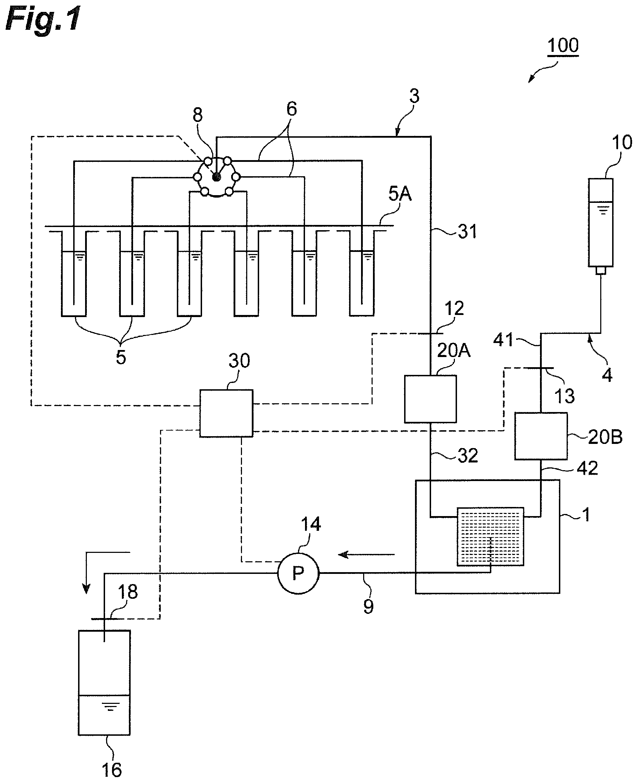

FIG. 1 is a view illustrating a configuration of a cell capturing apparatus according to the embodiment. The cell capturing apparatus refers to an apparatus capturing cells included in blood by filtering the blood being a test liquid with a filter. Identification of the cells and count of the number of individual cells are carried out by staining the cells captured with a filter using a processing liquid such as a staining liquid. In the following embodiment, the case where blood is the test liquid and the cells to be the capture target are Circulating Tumor Cells (CTCs) will be described. The present invention, however, is not limited to this example. Examples of the test liquid include blood and lymph, and examples of the specific cells being the capture target include rare cells represented by CTCs and cells in blood (red blood cells, white blood cells, platelets, and the like).

As illustrated in FIG. 1, a cell capturing apparatus 100 includes a cell capturing device 1 providing, inside the device, a filter capturing cells, a processing liquid channel 3 made of a soft tube supplying a processing liquid (a reagent) to the cell capturing device 1, and a test liquid channel 4 made of a soft tube supplying blood to the cell capturing device 1. On the upstream side of the processing liquid channel 3, a plurality of processing liquid storage containers 5 containing processing liquids (reagents) different from each other are provided. Examples of the processing liquid charged in the processing liquid storage container 5 include a staining liquid for staining cells, a cleaning liquid for cleaning the captured cells and the like on the filter, an immobilizing liquid for protecting cells from putrefaction, and permeation liquid for enabling the staining liquid to penetrate into the cell. The processing liquid storage containers 5 illustrated in FIG. 1 are sealed by a sealing member 5A. The constitution of the member, however, is not particularly limited.

A soft tube 6 is inserted into each of the processing liquid storage containers 5 to form an individual channel (processing liquid channel). These channels are connected to a selection valve 8. By rotating the selection valve 8, the processing liquid to be connected to the processing liquid channel 3 is selected and the soft tube 6 inserted into the processing liquid storage container 5 in which the selected processing liquid is contained and the processing liquid channel 3 are connected.

To the test liquid channel 4 connected to the cell capturing device 1, a blood storage container 10 (a test liquid storage container) containing blood including specific cells as the test liquid in the inside is connected. To the cell capturing device 1, the apparatus is configured not to supply the processing liquid and the blood at the same time but to supply either one of the two. Whether the processing liquid or the blood is supplied is controlled by switching valves 12 and 13 that are attached to the processing liquid channel 3 and the test liquid channel 4, respectively. For example, when the blood is supplied to the cell capturing device 1, the valve 12 is closed and the valve 13 is opened. As the valves 12 and 13, pinch valves, which shut off the flow in the soft tube by applying pressure to deform, can be used.

When either one liquid of the processing liquid or the blood is supplied to the cell capturing device 1, the liquid is supplied by sucking the target liquid by the drive of a pump 14 provided on a discharge channel 9 made of a soft tube on the downstream side of the cell capturing device 1. The pump 14 has a structure in which the flow rate of the liquid in the channel can be changed by rotational speed change. As the pump 14, for example, a peristaltic pump, which sequentially moves peristaltic points by pressure to the soft tube, can be used. By the drive of the pump 14, the liquid such as the processing liquid or the blood flow in the inside of the processing liquid channel 3 or the test liquid channel 4 in a direction toward the cell capturing device 1 and is supplied to the cell capturing device 1. The apparatus has a structure in which the liquid passing through the cell capturing device 1 flows into a waste liquid container 16 through the discharge channel 9. Thanks to these structures, the cells in the blood are captured by the filter provided on the channel in the cell capturing device 1 and stained with the staining liquid.

On the processing liquid channel 3 supplying the processing liquid and the test liquid channel 4 supplying the blood to the cell capturing device 1, the pre-processing unit carrying out pre-processing of the processing liquid is provided. Specifically, the processing liquid channel 3 supplying the processing liquid to the cell capturing device 1 includes a processing liquid channel 31 on the upstream side and a processing liquid channel 32 on the downstream side. A pre-processing unit 20A is disposed between the processing liquid channel 31 and the processing liquid channel 32. By this configuration, the processing liquid from the processing liquid channel 31 is introduced to the pre-processing unit 20A and the processing liquid through the pre-processing unit 20A is discharged to the processing liquid channel 32. The test liquid channel 4 supplying the blood to the cell capturing device 1 includes a test liquid channel 41 on the upstream side and a test liquid channel 42 on the downstream side. A pre-processing unit 20B is disposed between the test liquid channel 41 and the test liquid channel 42. By this configuration, the blood from the test liquid channel 41 is introduced to the pre-processing unit 20B and the blood through the pre-processing unit 20B is discharged to the test liquid channel 42. The configuration and the detail of the pre-processing units 20A and 20B will be described later.

Each of the parts is controlled by a control unit 30 (means for selection). Specifically, the selection valve 8, the valves 12, 13, and 18, and the pump 14 are driven by the instruction from the control unit 30. The control unit 30 is provided with a program input function for inputting a program that can control drive, stop, and the like of each of the parts. A drive mechanism in which each device is sequentially operated by the program inputted by the program input function is added. A line through which the liquid flows is selected by the control unit 30 (means for selection). Based on the selection result, instructions relating to opening and closing of the valves and drive of the pump (means for liquid supply) are issued from the control unit 30 to each of the parts.

### Cell Capturing Device

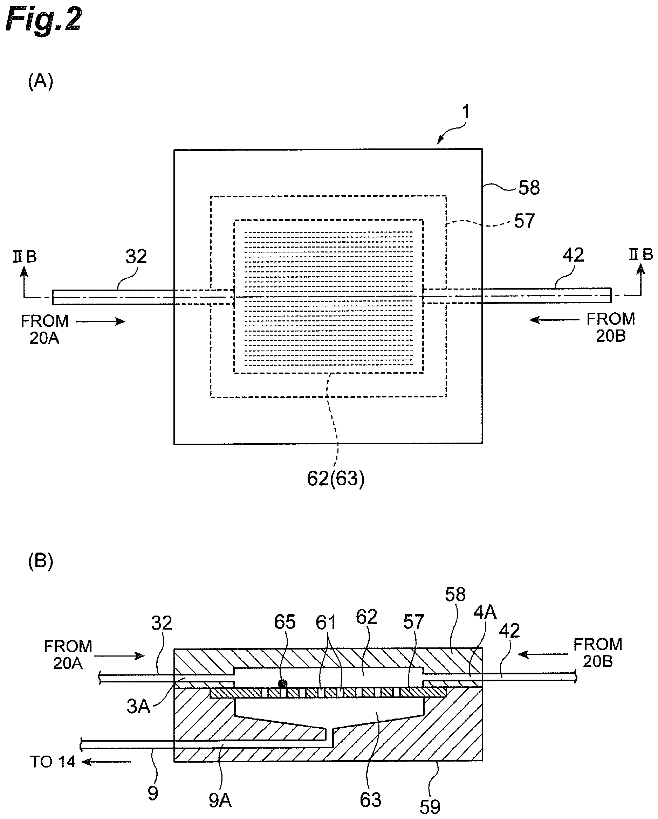

Subsequently, the cell capturing device 1 will be described with reference to FIG. 2. FIG. 2(A) is a top view illustrating the cell capturing device 1 and FIG. 2(B) is an arrow view taken from line IIB-IIB in FIG. 2(A).

The cell capturing device 1 has a structure in which a filter 57 having a plurality of through-holes 61 is sandwiched between a lid member 58 and a housing member 59. The filter 57 is disposed in a space formed in the inside of the lid member 58 and the housing member 59 when they are assembled. The filter 57 is made of a metal, for example, and is a filter in which the through-holes 61 are formed in a thickness direction of the filter.

From the viewpoint of workability and observability, the cell capturing device 1 preferably has a dimension of the length of one side of the lid member 58 in the top view of 10 mm to 100 mm, more preferably 15 mm to 70 mm, and further preferably 20 mm to 30 mm. From the viewpoint of the movable range in a height direction of an observation device, the cell capturing device 1 preferably has a thickness of 2 mm to 20 mm, more preferably 3 mm to 15 mm, and further preferably 5 mm to 10 mm.

Examples of the material of the metal used for the filter 57 include gold, silver, copper, aluminum, tungsten, nickel, chromium and alloys of these metals. The material of the metal, however, is not limited to these metals. The metal may be used singly or may be used as an alloy with other metals or the oxide of the metal for adding functionality. From the viewpoint of price or easy availability, nickel, copper, gold, and a metal containing these metals as a main component are preferably used. In particular, a metal containing nickel as a main component is preferably used. When the filter 57 is formed of the material containing nickel as the main component, the surface of nickel is preferably plated with gold. The gold plating can prevent oxidation of the filter surface and thus the adhesion to the specific cells (for example, CTCs) being the capture target becomes uniform and repeatability of data is improved.

The thickness of the filter 57 is preferably 3 µm to 100 µm. When the film thickness of the filter 57 is determined to be within the above range, the filter is easily handled and is also adequate for high-precision processing. The size of a region 62 where the through-holes 61 of the filter are provided is preferably 25 mm² to 1000 mm². The size is more preferably 25 mm² to 225 mm² and further preferably 25 mm² to 100 mm². A size of more than 1000 mm² results in an increase in a dead space, whereas a size of less than 25 mm² results in longer process time.

Examples of the opening shape of the through-holes of the filter 57 include ellipse, circle, rectangle, square, rounded rectangle, and polygon. The shape, however, is not limited to these shapes. The rounded rectangle means a rectangle having rounded edge corners. One example of the rounded rectangle includes a rectangle formed of two long sides having equal length and two semicircles and provided with semicircles on the outer side of the two short sides of the rectangle, where the midpoints of the short sides of the rectangle are determined to be the centers of the semicircles. The hole diameter of the through-holes of the filter 57 can be determined to be about 5.0 µm to about 15.0 µm. The hole diameter of the through-holes is more preferably 7.5 µm to 13.0 µm and further preferably 7.8 µm to 10.0 µm. The hole diameter of the through-holes means a diameter of the largest sphere that can pass through the through-holes. For example, the hole diameter of the through-hole having the opening shape of the rounded rectangle can be determined to be the length of the short side of the through-hole.

In the lid member 58 of the cell capturing device 1, an introduction channel 3A connected to the processing liquid channel 3 and an introduction channel 4A connected to the test liquid channel 4 formed of a soft tube are formed, and an introduction region 62 being a space communicating with the introduction channels 3A and 4A, formed on the upper side above the filter 57, and guiding the liquid to the through-holes 61 of the filter 57 is provided. In other words, the "introduction channel" in this embodiment refers to the introduction channels 3A and 4A provided in the inside of the cell capturing device 1 in the channels. The "processing liquid channel" and the "blood channel" refer to the processing liquid channel 3 and the test liquid channel 4 connected to the upstream side of the introduction channels 3A and 4A in the cell capturing device 1, respectively.

In the housing member 59 of the cell capturing device 1, a discharge region 63 being a space formed on the lower side of the filter 57 to form the center part more deeply than the peripheral part, and discharging the liquid that has passed through the through-holes 61 of the filter 57 to the outside is provided. In addition, in the housing member 59, a discharge channel 9A (discharge channel) communicating with the discharge region 63, connected to the discharge channel 9, and discharging the liquid in the discharge region 63 to the outside is provided. The through-holes 61 provided in the filter 57 sandwiched between the lid member 58 and the housing member 59 are determined to have such a size that the cells 65 to be the capture target cannot pass through.

The attaching positions of the processing liquid channel 32 (processing liquid channel 3), the test liquid channel 42 (test liquid channel 4), and the discharge channel 9 are not particularly limited to the positions illustrated in FIG. 2. In other words, the processing liquid channel 32 and the test liquid channel 42 are not required to be disposed opposite to each other. For example, the processing liquid channel 32 and the test liquid channel 42 may be connected in one of the four sides configuring the cell capturing device 1. The processing liquid channel 32 and the test liquid channel 42 may also be connected on the upper face. Similarly, the attaching position of the discharge channel 9 may be adequately changed. The attaching positions of the processing liquid channel 3, the test liquid channel 4, and the discharge channel 9 are changed depending on the introduction channels 3A and 4A and the discharge channel 9A in the cell capturing device 1 and thus the locations of the introduction channels 3A and 4A and the discharge channel 9A in the cell capturing device 1 may also be adequately changed. The number of the channels may also be adequately changed.

### Pre-processing Unit

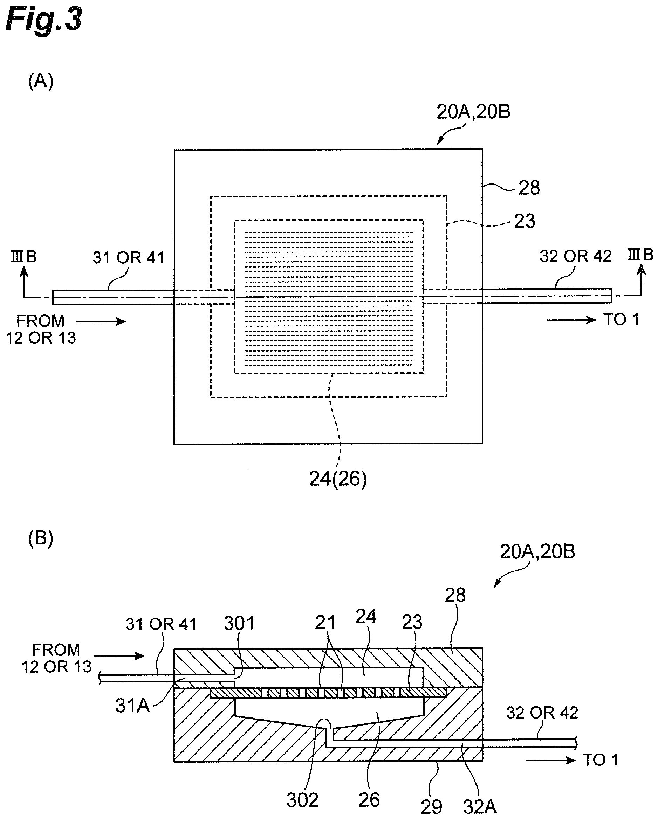

Subsequently, the pre-processing unit will be described with reference to FIG. 3. FIG. 3(A) is a top view illustrating the pre-processing unit and FIG. 3(B) is an arrow view taken from line IIIB-IIIB in FIG. 3(A). The inside of the pre-processing units 20A and 20B are configured by an introduction region 24 and a discharge region 26 sandwiching a foreign matter removal filter 23. The processing liquid channel 31 is connected to the introduction region 24 on the upper side of the pre-processing unit 20A and the processing liquid channel 32 is connected to the discharge region 26 of the lower part of the pre-processing unit 20A. Such a pre-processing unit 20A has a function that removes air bubbles and foreign matters in the processing liquid while the processing liquid is introduced from the processing liquid channel 31 and discharged to the processing liquid channel 32. The pre-processing unit 20B has a configuration similar to the configuration of the pre-processing unit 20A except that blood is introduced from the test liquid channel 41 instead of the processing liquid channel 31 and the blood is discharged to the test liquid channel 42.

From the view point of workability and observability, the pre-processing unit 20A preferably has a dimension of the length of one side of the lid member 58 in the top view of 10 mm to 100 mm, more preferably 15 mm to 70 mm, and further preferably 20 mm to 30 mm. From the viewpoint of the movable range in a height direction of an observation device, the pre-processing unit 20A preferably has a thickness of 2 mm to 20 mm, more preferably 3 mm to 15 mm, and further preferably 5 mm to 10 mm. The dimension and the thickness of the pre-processing unit 20B is the same as the dimension and the thickness of the pre-processing unit 20A.

The pre-processing units 20A and 20B can remove not only the air bubbles but also the foreign matters in the processing liquid by the structure in which a foreign matter removal filter 23 having a plurality of through-holes 21 is sandwiched between a lid member 28 and a housing member 29. The planar shapes of the lid member 28 and the housing member 29 may be a rectangle or may be a circle similarly to the cell capturing device 1, and are not particularly limited.

The foreign matter removal filter 23 is disposed in a space formed in the inside of the lid member 28 and the housing member 29 when they are assembled. The foreign matter removal filter 23 is a filter forming the through-holes 21 in a thickness direction of the filter. Examples of the shape of the through-holes 21 of the foreign matter removal filter 23 include a shape similar to the shape exemplified as the through-holes of the cell capturing device. However, the shape is not limited to these shapes. Similarly to the through-holes of the cell capturing device, the hole diameter of the through-holes means a diameter of the largest sphere that can pass through the through-holes. The size of the through-holes 21 provided in the foreign matter removal filter 23 is required to be a size large enough for all types of the processing liquids to pass. The size is preferably such a size that any types of the processing liquids can pass through, that is, load is not applied at the time of passing. In addition, the size of the through-holes 21 of the foreign matter removal filter 23 in the pre-processing unit 20A through which the processing liquid passes is preferably equal to or smaller than the through-holes of filter 57 in the cell capturing device. Specifically, the hole diameter of the through-holes 21 of the foreign matter removal filter 23 in the pre-processing unit 20A through which the processing liquid flows is preferably 1 µm to 5 µm and more preferably 2 µm to 5 µm. By such a configuration, the foreign matters can be surely removed at the pre-processing unit 20A of the preceding stage, when the foreign matters are mixed in the processing liquid. By contrast, the size of the through-holes 21 of the foreign matter removal filter 23 in the pre-processing unit 20B through which blood passes is preferably sufficiently larger than the size of the cells being the capture target and larger than the size of the hole of the filter 57 in the cell capturing device. Specifically, the hole diameter of the through-holes 21 of the foreign matter removal filter 23 in the pre-processing unit 20B through which the processing liquid flows is preferably 15 µm to 100 µm, more preferably 18 µm to 100 µm, and further preferably 20 µm to 50 µm. Although the material of the foreign matter removal filter 23 is not particularly limited, the thickness can be thinner by taking advantage of its high rigidity when the material is a metal.

Examples of the material of the metal used for the foreign matter removal filter 23 include gold, silver, copper, aluminum, tungsten, nickel, chromium and alloys of these metals. The material of the metal, however, is not limited to these metals. The metal may be used singly or may be used as an alloy with other metals or the oxide of the metal for adding functionality. From the viewpoint of price or easy availability, nickel, copper, gold, and a metal containing these metals as a main component are preferably used. In particular, a metal containing nickel as a main component is preferably used. When the foreign matter removal filter 23 is formed of the material containing nickel as the main component, the surface of nickel is preferably plated with gold. The gold plating can prevent oxidation of the filter surface and thus the foreign matter removal function can be maintained high. The filter surface is preferably covered with a polymer that is harmless to cells. In this case, however, adhesion to the cells cannot be required.

The thickness of the foreign matter removal filter 23 is preferably 3 µm to 100 µm. When the film thickness is determined to be within the above range, the filter is easily handled and is also adequate for high-precision processing. The size of the region 24 where the through-holes 21 of the filter are provided is preferably 36 mm² to 1500 mm². The size of the region is more preferably 36 mm² to 750 mm² and further preferably 36 mm² to 225 mm². A size of more than 1500 mm² results in an increase in dead space, whereas a size of less than 36 mm² results in longer process time.

In the lid member 28 of the pre-processing unit 20A, a channel 31A (an introduction channel) connected to the processing liquid channel 31 is formed and the introduction region 24 being a space communicating with the channel 31A, formed on the upper side above the foreign matter removal filter 23, and guiding the processing liquid to the through-holes 21 of the foreign matter removal filter 23 is provided. In the case of the pre-processing unit 20B, the test liquid channel 41 is connected to the upstream side of a channel 41A.

In the housing member 29 of the pre-processing unit 20A, the discharge region 26 being a space formed on the lower side of the foreign matter removal filter 23 to form the center part more deeply than the peripheral part, and discharging the liquid that has passed through the through-holes 21 of the foreign matter removal filter 23 to the outside is provided. In addition, in the housing member 29, a channel 32A (a discharge channel) communicating with the discharge region 26, connected to the processing liquid channel 32, and discharging the liquid in the discharge region 26 to the outside is provided at the center of the discharge region 26. In the case of the pre-processing unit 20B, the test liquid channel 42 is connected to the downstream side of the channel 32A. The bottom part of the discharge region 26 is gradually recessed from the edge part to the center and the channel 32A is provided at the center, which is the deepest part. This configuration allows the processing liquid from the channel 32A to be suitably discharged. In the pre-processing unit 20A, the space formed by the introduction region 24 and the discharge region 26 functions as an air bubble accumulated region having a larger diameter than the diameter of the processing liquid channel 3 (the processing liquid channels 31 and 32) through which the processing liquid passes. Similarly, in the pre-processing unit 20B, the space formed by the introduction region 24 and the discharge region 26 functions as an air bubble accumulated region having a larger diameter than the diameter of the test liquid channel 4 (the test liquid channels 41 and 42) through which the blood passes. From the viewpoint of repeatability, the sizes of the channels in the whole cell capturing apparatus 100 preferably basically have similar sizes except the air bubble accumulated region (the space formed by the introduction region 24 and the discharge region 26) of the pre-processing unit 20A and the pre-processing unit 20B and the space (the space formed by the introduction region 62 and the discharge region 63) inside of the cell capturing device 1. The diameter of the air bubble accumulated region means a diameter of the cross-section viewed in the moving direction of the processing liquid in the air bubble accumulated region.

As the pre-processing unit 20A (and/or the pre-processing unit 20B), the cell capturing device 1 illustrated in FIG. 2 can be used. The cell capturing device 1 illustrated in FIG.2 is provided with the introduction channels 3A and 4A in the lid member 58 being the upstream side and to be able to be connected to the two channels different from each other. In contrast, in the pre-processing unit 20A illustrated in FIG. 3, the lid member 28 on the upstream side is provided with one channel 31A. Therefore, an edge part on the side connected to the outside channel (for example, the processing liquid channel 3) of one channel (for example, the introduction channel 3A) of the cell capturing device 1 illustrated in FIG. 2 is sealed to form the cell capturing device 1 having one channel being able to connect to the outside in the lid member 58, whereby the cell capturing device 1 can be modified to an configuration similar to the configuration of the pre-processing unit 20A illustrated in FIG. 3. As described above, the pre-processing unit 20A (and/or the pre-processing unit 20B) can be produced by using the lid member 28 and the housing member 29 having the same shapes as the lid member 58 and the housing member 59 of the cell capturing device 1, respectively. This can reduce the production cost compared with the case, for example, where the pre-processing unit 20A (and/or the pre-processing unit 20B) having a shape different from the cell capturing device 1 is produced. Namely, "the same shape" described here means a shape in which the location and the number of the channels are the same and the dimensions of each part when formed using a casting vary within the range of fluctuation. The error that is allowable as the "fluctuation" is within ± 3 µm.

By providing the lid member 28 in the cell capturing apparatus 100 so that the lid member 28 is provided on the upper side of the housing member 29 in a height direction, in the pre-processing unit 20A, an edge part 301 on the introduction region 24 side of the channel 31 A being the introduction port of the processing liquid to the inside space (the introduction region 24 and the discharge region 26) is provided above an edge part 302 on the discharge region 26 side of the channel 32A being the discharge port. By this configuration, the processing liquid passing through the air bubble accumulated region configured from the introduction region 24 and the discharge region 26 is introduced to the introduction region 24 from the upper introduction port and discharged to the outside from the discharge port provided under the discharge region 26. Here, the inner pressure of the processing liquid or the test liquid that has reached the air bubble accumulated region is dropped at once and thus the air bubbles dissolved in the liquid are separated and the separated air bubbles tend to accumulate. This allows the effect of air bubble removal from the processing liquid to be improved. The air bubbles separated from the processing liquid is moved to the upper side of the introduction region 24 and accumulates. By passing the processing liquid through the through-holes 21 of the foreign matter removal filter 23, the foreign matters that cannot pass through the through-holes 21 are captured by the foreign matter removal filter 23. This allows the processing liquid from which air bubbles and foreign matters are removed to be discharged from the discharge region 26 to the processing liquid channel 32 in the subsequent stage through the channel 32A. As the positional relation between the edge part 301 on the introduction region 24 side of the channel 31A being the introduction port and the edge part 302 on the discharge region 26 side of the channel 32A being the discharge port, the lower edge of the edge part 301 on the introduction port side is preferably provided above the upper edge of the edge part 302 on the discharge port side. Specifically, the lower edge of the edge part 301 on the introduction port side is preferably provided 0.1 mm to 5 mm higher, more preferably 0.1 mm to 2.5 mm higher, and further preferably 0.1 mm to 1.5 mm higher than the upper edge of the edge part 302 on the discharge port side. By providing the lower edge of the edge part 301 on the introduction port side 0.1 mm to 5 mm higher than the upper edge of the edge part 302 on the discharge port side, the air bubbles and the foreign matters can be adequately removed.

As described above, by providing the pre-processing unit 20A on the processing liquid channel, air bubbles accumulate in the introduction region 24 inside of the pre-processing unit 20A and thus the air bubbles are removed while the processing liquid moves through the introduction region 24 and the discharge region 26, even when the air bubbles are contained in the processing liquid. By providing the foreign matter removal filter 23, at least a part of the foreign matters (the foreign matters that cannot pass through the through-holes 21) contained in the processing liquid can be captured and thus the processing liquid from which the foreign matters are removed can be supplied to the cell capturing device 1 at the subsequent stage. Similarly, by providing the pre-processing unit 20B on the blood channel, air bubbles are accumulated in the introduction region 24 inside of the pre-processing unit 20B and thus the air bubbles are removed while the blood moves through the introduction region 24 and the discharge region 26, even when the air bubble are contained in the blood. By providing the foreign matter removal filter 23, at least a part of the foreign matters contained in blood (the foreign matters that cannot pass through the through-holes 21; for example, deformed white blood cells that cannot pass through the through-holes 21) can be captured and thus the blood from which the foreign matters are removed can be supplied to the cell capturing device 1 at the subsequent stage.

The cross-sectional area viewed in the moving direction of the processing liquid in the air bubble accumulated region is preferably sufficiently larger than the cross-sectional areas of the processing liquid channels 31 and 32 connected to the air bubble accumulated region, and, for example, is preferably 10 times or more larger than the cross-sectional areas of the processing liquid channels 31 and 32. By providing the air bubble accumulated region having a larger cross-sectional area than the cross-sectional areas of the processing liquid channels 31 and 32, the inner pressure of the processing liquid that has reached the air bubble accumulated region is reduced. This results in separating the air bubbles from the processing liquid to increase opportunity to contact other air bubbles. As a result, the resultant air bubbles easily accumulate as air bubbles. The cross-sectional area viewed in the moving direction of the processing liquid in the air bubble accumulated region can be determined as the area of the introduction region 24 in the top view of the pre-processing unit 20A.

When the cross-sectional area viewed in the moving direction of the processing liquid in the cell capturing device 1 is determined to be the area of the introduction region 62 in the top view of the cell capturing device 1, the cross-sectional area viewed in the moving direction of the processing liquid in the air bubble accumulated region in the pre-processing unit 20A is preferably equal to or larger than the cross-sectional area viewed in the moving direction of the processing liquid in the cell capturing device 1. This allows the air bubbles in the processing liquid to be sufficiently accumulated in the air bubble accumulated region of the pre-processing unit 20A and allows the structure in which air bubbles are difficult to be generated in the cell capturing device 1 on the downstream side to be formed.

Similarly to the pre-processing unit 20A, in the pre-processing unit 20B, the cross-sectional area viewed in the moving direction of the test liquid in the air bubble accumulated region is preferably sufficiently larger than the cross-sectional area of the test liquid channels 41 and 42 connected to the air bubble accumulated region, and, for example, is preferably 10 times or more larger than the cross-sectional areas of the processing liquid channels 41 and 42. When the cross-sectional area viewed in the moving direction of the test liquid in the cell capturing device 1 is determined to be the area of the introduction region 62 in the top view of the cell capturing device 1, the cross-sectional area viewed in the moving direction of the test liquid in the air bubble accumulated region in the pre-processing unit 20B is preferably equal to or larger than the cross-sectional area viewed in the moving direction of the test liquid in the cell capturing device 1.

### Cell Capturing Device Provided with Pre-processing Unit

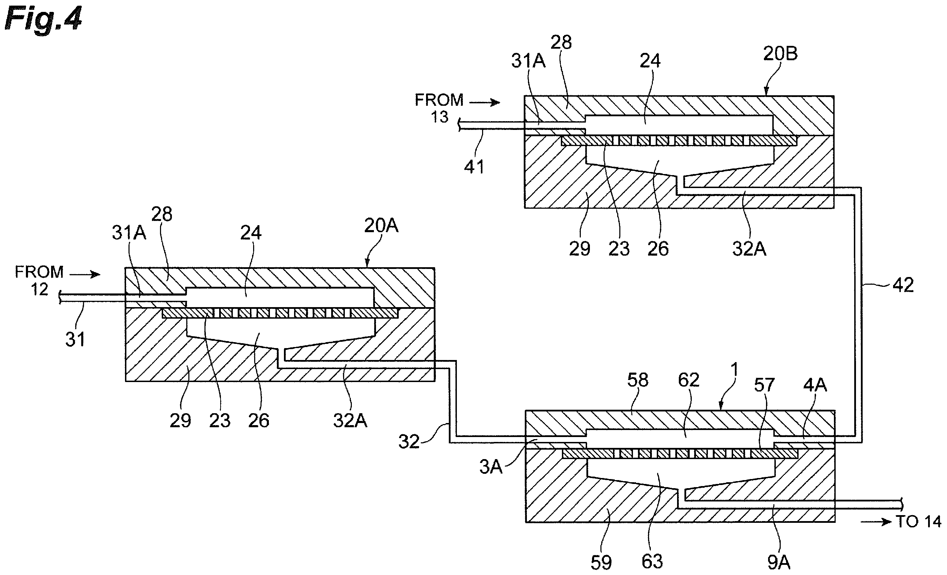

In FIG. 4, a configuration in which the cell capturing device 1 illustrated in FIG. 2, the pre-processing unit 20A illustrated in FIG. 3,and the pre-processing unit 20B having a configuration similar to the configuration of the pre-processing unit 20A are connected is illustrated. The processing liquid supplied from the processing liquid storage container 5 is supplied to the pre-processing unit 20A through the processing liquid channel 31 on the downstream side from the valve 12. In the pre-processing unit 20A, the processing liquid is introduced from the channel 31A to the introduction region 24 and moves to the discharge region 26 through the foreign matter removal filter 23. At this time, the air bubbles in the processing liquid accumulate in the upper side of the introduction region 24 and thus the air bubbles are removed from the processing liquid. The foreign matters that cannot pass through the foreign matter removal filter 23 are captured by the foreign matter removal filter 23 and thus the foreign matters in the processing liquid are removed. The processing liquid from which the air bubbles and the foreign matters are removed is discharged from the channel 32A to the outside of the pre-processing unit 20A and supplied to the cell capturing device 1 through the processing liquid channel 32. The processing liquid from which the air bubbles are removed is discharged from the discharge channel 9A to the outside of the cell capturing device 1 through the introduction channel 3A in the cell capturing device 1 and through the introduction region 62, the filter 57, and the discharge region 63.

The blood supplied from the blood storage container 10 is supplied to the pre-processing unit 20B through the test liquid channel 41 on the downstream side from the valve 13. In the pre-processing unit 20B, the blood is introduced from the channel 41A to the introduction region 24 and moves to the discharge region 26 through the foreign matter removal filter 23. At this time, the air bubbles in the blood accumulate in the upper side of the introduction region 24 and thus the air bubbles are removed from the blood. The foreign matters that cannot pass through the foreign matter removal filter 23 are captured by the foreign matter removal filter 23 and thus the foreign matters in the blood are removed. The blood from which the air bubbles and the foreign matters are removed is discharged from the channel 32A to the outside of the pre-processing unit 20B and supplied to the cell capturing device 1 through the test liquid channel 42. The processing liquid from which the air bubbles are removed is discharged from the discharge channel 9A to the outside of the cell capturing device 1 through the introduction channel 3A in the cell capturing device 1 and through the introduction region 62, the filter 57, and the discharge region 63.

For the pre-processing unit, the cell capturing device, and the cell capturing device provided with a pre-processing unit according to the embodiment of the present invention, these devices and the channels provided for connecting these devices are preferably used in a state that the inside of the devices and the channels are filled with cleaning solution, saline, deionized water, or the like.

As described above, the embodiment of the present invention is described. The cell capturing apparatus, the cell capturing device provided with a pre-processing unit, and the pre-processing unit according to the present invention are not limited to the above embodiment and various modifications can be carried out.

For example, in the above embodiment, the configuration in which the pre-processing unit 20A is provided on the processing liquid channel 3 and the pre-processing unit 20B is provided on the test liquid channel 4 is described. The configuration, however, may be a configuration in which either one of the pre-processing units 20A or 20B is provided. In this case, the air bubbles and the foreign matters are also suitably removed from the liquid flowing through the channels on the side where the pre-processing unit is provided and thus the process relating to capturing cells can be carried out with good repeatability.

In the above embodiment, the case that the pre-processing unit 20A (20B) has a configuration similar to the configuration of the cell capturing device 1 illustrated in FIG. 2 is described. The pre-processing unit 20A (20B), however, may have a configuration different from the cell capturing device 1. For example, the lid member 28 and the housing member 29 are not required to be included and may be integrally molded.

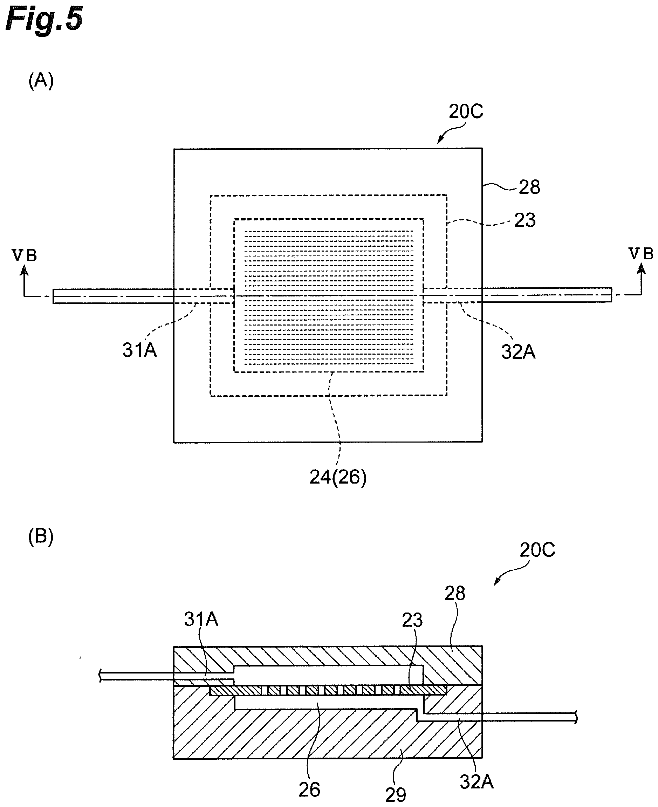

Here, modified examples of the pre-processing unit will be described with reference to the FIG. 5 to FIG. 10. First, FIG. 5(A) is a top view illustrating a pre-processing unit 20C and FIG. 5(B) is an arrow view taken from line VB-VB in FIG. 5(A). In the pre-processing unit 20C illustrated in FIG. 5, the shape of the discharge region 26 in the housing member 29 on the downstream side from the foreign matter removal filter 23 is modified compared with the pre-processing unit 20A, which is described as the embodiment. Specifically, the center part is not sloped so that the center part is deep and thus the bottom part is horizontal. The channel 32A for discharge is provided at the edge part of the bottom part.

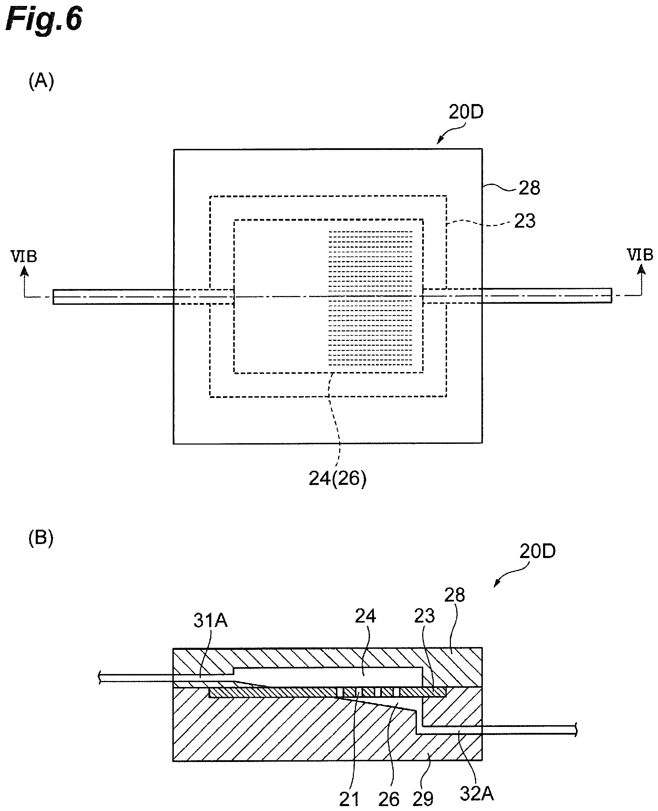

Subsequently, FIG. 6(A) is a top view illustrating a pre-processing unit 20D and FIG. 6(B) is an arrow view taken from line VIB-VIB in FIG. 6(A). Compared with the pre-processing unit 20A, the pre-processing unit 20D illustrated in FIG. 6 is different in that the bottom surface is sloped from the channel 31 A on the introduction region 24 side to the channel 32A on the discharge region 26 side. Consequently, the area of the region where the through-holes 21 of the foreign matter removal filter 23 is provided (the area where the liquid can pass through) is smaller than that of the pre-processing unit 20A. The liquid passing through the through-holes 21 of the foreign matter removal filter 23, however, can be suitably discharged from the channel 32A.

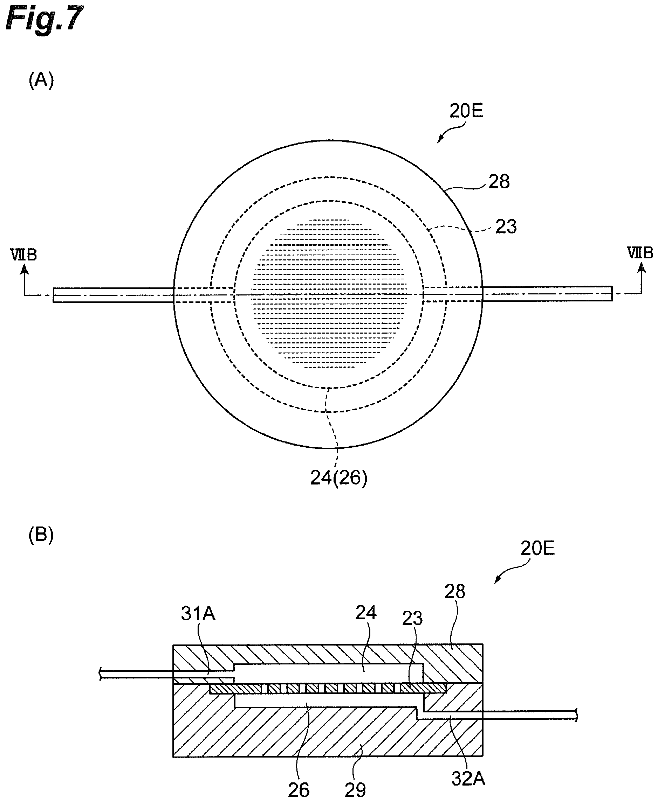

FIG. 7(A) is a top view illustrating a pre-processing unit 20E and FIG. 7(B) is an arrow view taken from line VIIB-VIIB in FIG. 7(A). The pre-processing unit 20E illustrated in FIG. 7 has an inside structure similar to the inside structure of the pre-processing unit 20C but the outer shape formed by the lid member 28 and the housing member 29 is a circular shape. The shape of inside of the introduction region 24, the discharge region 26, and the region where the through-holes 21 of the foreign matter removal filter 23 provided therebetween (the region where the liquid can pass through) are formed are also formed as the circular shape when the shape is seen from the top face. As described above, the shapes of the introduction region 24, the discharge region 26, and the region where the through-holes 21 of the foreign matter removal filter 23 are provided can be adequately modified.

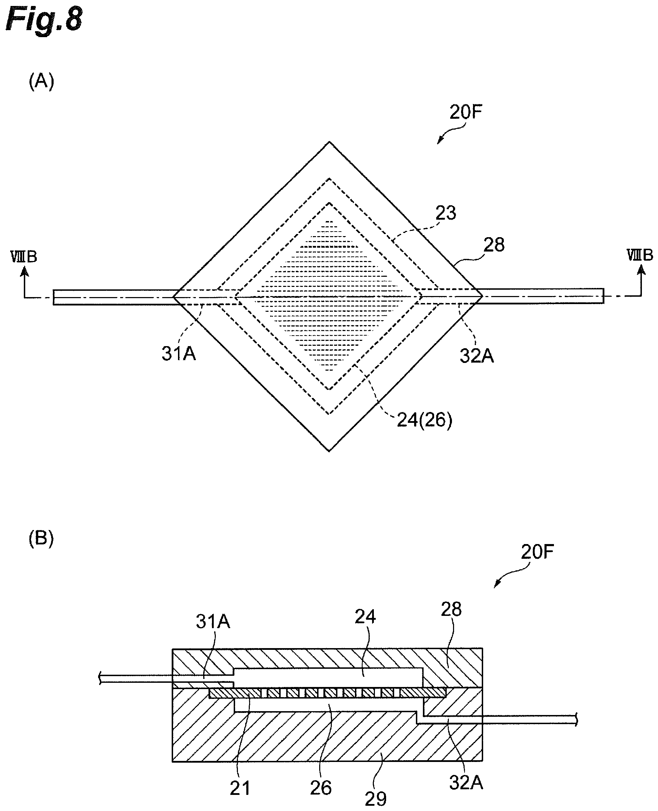

FIG. 8(A) is a top view illustrating a pre-processing unit 20F and FIG. 8(B) is an arrow view taken from line VIIIB-VIIIB in FIG. 8(A). Although the pre-processing unit 20F illustrated in FIG. 8 has an outer shape of a quadrilateral shape, the positions where the channels 31A and 32A are attached are different from those of the pre-processing unit 20C and other pre-processing units. More specifically, the pre-processing unit 20C has the configuration in which, in the introduction region 24 and the discharge region 26 of quadrilateral shapes, the channel 31 A is provided near the center of the one side of the quadrilateral shape and the channel 32A is provided near the center of the one side opposed to the side where the channel 31A is provided (refer to FIG. 5(A) and the like). In contrast, in the pre-processing unit 20F, the channels 31 A and 32A are provided at the vertices parts of the opposed comers in the introduction region 24 and the discharge region 26 of the quadrilateral shape.

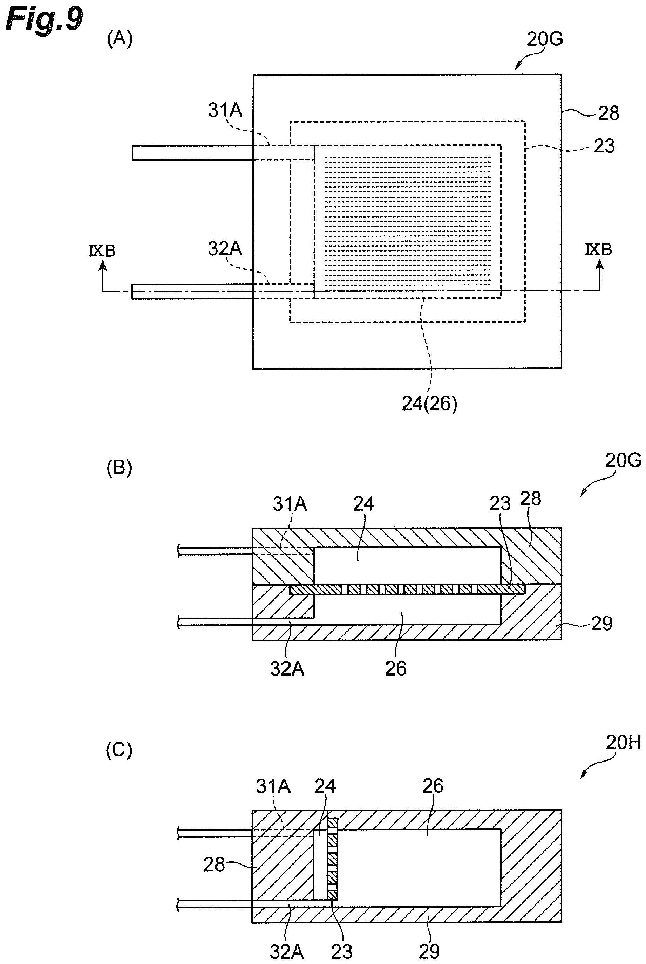

FIG. 9(A) is a top view illustrating a pre-processing unit 20G and FIG. 9(B) is an arrow view taken from line IXB-IXB in FIG. 9(A). Although the pre-processing unit 20G illustrated in FIG. 9(A) and FIG. 9(B) has an outer shape of a quadrilateral shape, the positions where the channels 31 A and 32A are attached are different from those of the pre-processing unit 20C and other pre-processing units. In the pre-processing unit 20G, the channel 31 A is provided in the introduction region 24 and the discharge region 26 of the quadrilateral shape on one edge side of one side of the quadrilateral shape and the channel 32A is provided on the other edge side. As illustrated in the above modified examples, the positions where the channels 31A and 32A are provided are not particularly limited. For example, as with the pre-processing unit 20G illustrated in FIG. 9, both of the channels 31A and 32A may be provided on one side of the introduction region 24 and the discharge region 26 of the quadrilateral shape or each channel may be provided in sides different from each other. As with the pre-processing unit 20A and the like, when the channel 31 A and the channel 32A are provided in the opposed sides, the channels 31 A and 32A may not be provided on the same line (refer to IIIB-IIIB of FIG. 3).

FIG. 9(C) is view illustrating a pre-processing unit 20H that has the position to which the foreign matter removal filter 23 is attached, the position being changed from that of the pre-processing unit 20G. This view corresponds to FIG. 9(B). In contrast to the pre-processing units 20A to 20G to which the foreign matter removal filter 23 is attached in a horizontal direction, the filter is attached to the pre-processing unit 20H illustrated in FIG. 9(C) in a vertical direction. At this time, the upstream side (the left side in the view) from the foreign matter removal filter 23 is determined to be the introduction region 24, while the downstream side (the right side in the view) from the foreign matter removal filter 23 is determined to be the discharge region 26. The shapes of lid member 28 and the housing member 29 are changed so as not to communicate the introduction region 24 and the discharge region 26. In the case of the pre-processing unit 20H, the configuration in which the edge part of the channel 32A on the discharge region 26 side supports the one edge side of the foreign matter removal filter 23 is illustrated as one example. As described above, the attached direction of the foreign matter removal filter 23 can also be adequately changed in the pre-processing unit. The shapes of the lid member 28 and the housing member 29 can also be changed in accordance with, for example, the position to which the foreign matter removal filter 23 is attached.

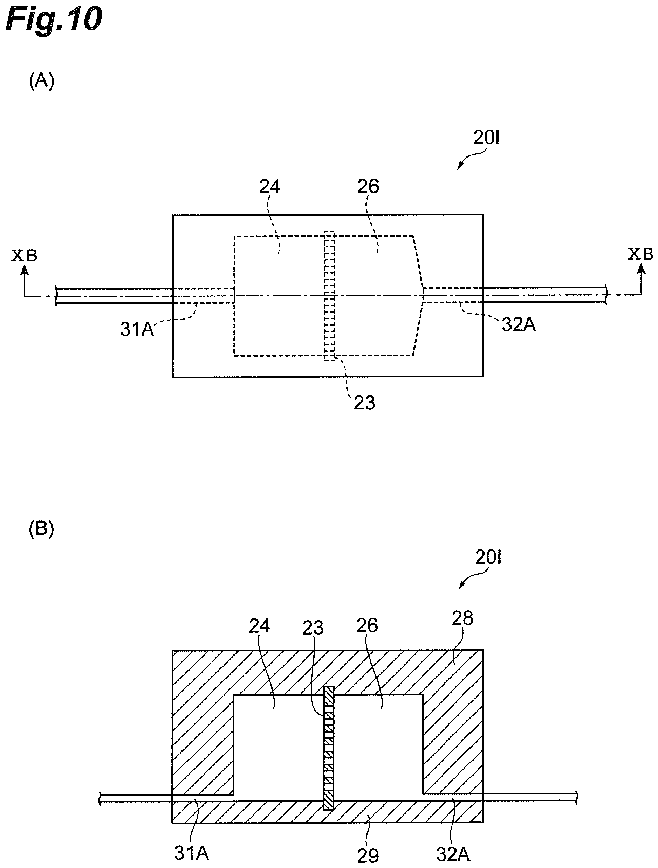

FIG. 10 is a view illustrating a pre-processing unit 20I that has the position to which the foreign matter removal filter 23 is attached, the position being changed from that of the pre-processing unit 20G. FIG. 10(A) is a top view illustrating the pre-processing unit 20I and FIG. 10(B) is an arrow view taken from line XB-XB in FIG. 10(A). The inside of the pre-processing unit 20I includes the introduction region 24 and the discharge region 26 sandwiching the foreign matter removal filter 23. When the pre-processing unit 20I is provided on the processing liquid channel 3, the processing liquid channel 31 is connected to the introduction region 24 of the pre-processing unit 20I and the processing liquid channel 32 is connected to the discharge region 26 of the pre-processing unit 20I. In this case, the pre-processing unit 20I has a function in which the air bubbles and the foreign matters in the processing liquid are removed while the processing liquid is introduced from the processing liquid channel 31 and discharged to the processing liquid channel 32. When the pre-processing unit 20I is provided on the test liquid channel 4, blood is introduced from the test liquid channel 41 connected to the introduction region 24 of the pre-processing unit 20I and the blood is discharged to the test liquid channel 42 connected to the discharge region 26 of the pre-processing unit 20I.

From the view point of workability and observability, the pre-processing unit 20I preferably has a dimension of the length of one side of the lid member 28 in the top view of 10 mm to 100 mm, more preferably 15 mm to 70 mm, and further preferably 20 mm to 30 mm. From the viewpoint of the movable range in a height direction of an observation device, the pre-processing unit 20I preferably has a thickness of 2 mm to 20 mm, more preferably 3 mm to 15 mm, and further preferably 5 mm to 10 mm.

The pre-processing unit 20I can remove not only the air bubbles but also the foreign matters in the processing liquid by the structure in which a foreign matter removal filter 23 having a plurality of through-holes 21 is sandwiched between a lid member 28 and a housing member 29. The planar shape of the lid member 28 and the housing member 29 may be a rectangle or may be a circle similarly to the cell capturing device 1, and is not particularly limited.

The foreign matter removal filter 23 is disposed in a space formed in the inside of the lid member 28 and the housing member 29 when they are assembled. The foreign matter removal filter 23 is a filter provided with the through-holes in a thickness direction of the filter. The hole diameter of the through-holes in the foreign matter removal filter 23 is adequately selected whether the pre-processing unit 20I is used for the processing liquid or used for the test liquid. Similarly to the pre-processing unit 20H, the foreign matter removal filter 23 is attached in a vertical direction. At this time, the upstream side (the left side in the view) from the foreign matter removal filter 23 is determined to be the introduction region 24, while the downstream side (the right side in the view) from the foreign matter removal filter 23 is determined to be the discharge region 26. The shapes of lid member 28 and the housing member 29 are changed so as not to communicate the introduction region 24 and the discharge region 26.

In the pre-processing unit 20I, the channels 31A and 32A are provided at the bottom part of the introduction region 24 as illustrated in FIG. 10(B). When such a pre-processing unit 20I is applied for the processing liquid channel 3, the inner pressure of the processing liquid is reduced to separate the air bubbles from the processing liquid in the pre-processing unit 20I when the processing liquid has reached the introduction region 24 from the channel 31A. The specific gravity of the air bubbles is smaller than that of the liquids and thus the separated air bubbles themselves move to the upper part of the introduction region 24 to accumulate in the upper part. By contrast, the processing liquid flows through the lower part of the introduction region 24 to remove the foreign matters by the foreign matter removal filter 23, and thereafter flows from the channel 32A to the channel at the subsequent stage (for example, the channel 32). As illustrated in FIG. 10(A), the pre-processing unit 20I has a tilted side wall of the discharge region 26 so that the processing liquid easily flows to the channel 32A, when the pre-processing unit 20I is seen from the top face. Consequently, the processing liquid passing through the foreign matter removal filter 23 can be suitably discharged from the channel 32A to the subsequent stage. As described above, the attached direction of the foreign matter removal filter 23 can also be adequately changed in the pre-processing unit. The shapes of the lid member 28 and the housing member 29 can also be changed in accordance with, for example, the position to which the foreign matter removal filter 23 is attached.

### Examples

Hereinafter, the present invention will be more specifically described with reference to Examples and Comparative Examples. The present invention, however, is not limited to these Examples.

### Example 1

As Example 1, air bubble removal effect by the pre-processing unit 20A was ascertained using the pre-processing unit 20A (provided with the foreign matter removal filter 23) and the cell capturing device 1 illustrated in FIG. 4. At this time, the inner diameter of the processing liquid channels 31 and 32 had a diameter of 1 mm (a cross-sectional area of 0.8 mm²) and the inner diameter of the channels 31A, 32A, and 3A of the pre-processing unit 20 and the cell capturing device 1 had a diameter of 0.8 mm (a cross-sectional area of 0.5 mm²). The volume of the air bubble accumulated region configured by the introduction region 24 and the discharge region 26 was 79 mm³ (a cross-sectional area of 56 mm²: a square shape having a side length of 7.5 mm, and a height of 1.4 mm). As the foreign matter removal filter 23, a metal filter having a hole diameter of 8×100 µm and a hole opening ratio of 8.7% was used. For the pre-processing unit 20A, the inside of the channels leading to the cell capturing device 1 was previously filled with degassed pure water. When water mixed with air flowed as a processing liquid to such a pre-processing unit 20A, the air bubbles mixed in the processing liquid accumulated in the introduction region 24 and did not flow into the channel 32A on the discharge side. As a result, the processing liquid from which the air bubbles were removed was supplied to the cell capturing device 1. When the cell capturing device 1 was filled with the processing liquid passing through the foreign matter removal filter 23 and was observed by a microscope, the foreign matter larger than the size of the through-holes 21 of the foreign matter removal filter 23 were not ascertained. Consequently, the cell capturing device 1 was able to be observed well.

### Example 2

As Example 2, air bubble removal effect by the pre-processing unit 20A was ascertained using the pre-processing unit 20A (provided with the foreign matter removal filter 23) and the cell capturing device 1 illustrated in FIG. 4. At this time, the inner diameter of the processing liquid channels 31 and 32 had a diameter of 1 mm (a cross-sectional area of 0.8 mm²) and the inner diameter of the channels 31A, 32A, and 3A of the pre-processing unit 20 and the cell capturing device 1 had a diameter of 0.8 mm (a cross-sectional area of 0.5 mm²). The volume of the air bubble accumulated region configured by the introduction region 24 and the discharge region 26 was 79 mm³ (a cross-sectional area of 56 mm²: a square shape having a side length of 7.5 mm, and a height of 1.4 mm). As the foreign matter removal filter 23, a metal filter having circular holes having a diameter of 35 µm and a hole opening ratio of 30% was used. For the pre-processing unit 20A, the inside of the channels leading to the cell capturing device 1 was previously filled with degassed pure water. When blood flowed as a test liquid mixed with air bubbles to such a pre-processing unit 20, the air bubbles mixed in the test liquid accumulated in the introduction region 24 and did not flow into the channel 32A on the discharge side. As a result, the test liquid from which the air bubbles were removed was supplied to the cell capturing device 1. When the cell capturing device 1 was filled with the test liquid passing through the foreign matter removal filter 23 and was observed by a microscope, the foreign matter larger than the size of the through-holes 21 of the foreign matter removal filter 23 were not ascertained and the agglomerates caused by the deterioration with time were removed. Consequently, the whole observation field was clear and the captured cells were able to be observed well.

### Comparative Example 1

As Comparative Example 1, air bubble removal effect by the pre-processing unit 20A was ascertained using the pre-processing unit 20A (however, not provided with the foreign matter removal filter 23) and the cell capturing device 1 illustrated in FIG. 4. At this time, the inner diameter of the processing liquid channels 31 and 32 had a diameter of 1 mm (a cross-sectional area of 0.8 mm²) and the inner diameter of the channels 31A, 32A, and 3A of the pre-processing unit 20 and the cell capturing device 1 had a diameter of 0.8 mm (a cross-sectional area of 0.5 mm²). The volume of the air bubble accumulated region configured by the introduction region 24 and the discharge region 26 was 79 mm³ (a cross-sectional area of 56 mm²/a square shape having a side length of 7.5 mm). For the pre-processing unit 20A, the inside of the channels leading to the cell capturing device 1 was previously filled with degassed pure water. When water mixed with air flowed as a processing liquid to such a pre-processing unit 20A, it was ascertained that although the air bubbles mixed in the processing liquid accumulated in a certain amount in the introduction region 24, a part of the air bubble did not flow into the channel 32A on the discharge side. As a result, air bubbles were supplied to the cell capturing device 1. When the cell capturing device 1 after the processing was observed by a microscope, a substantial amount of the foreign matters derived from the processing liquid was ascertained in addition to the captured cells and thus the foreign matters were difficult to be distinguished from the captured cells. In addition, some foreign matters emitted strong fluorescence having high light intensity and the observation of the captured cells around the foreign matters was disturbed.

### Comparative Example 2

As Comparative Example 2, at the previous stage of the cell capturing device 1, a T-branch pipe having the same channel cross-sectional area (a cross-sectional area of 0.8 mm²) as the channel cross-sectional area of the processing liquid channels 31 and 32 was attached instead of the pre-processing unit, where each of the two pipe parts in the T-branch pipe was connected to processing liquid channels 31 and 32, and the pipe part to which the channel was not connected was attached as the pipe part is directed upward and the edge part of the pipe part was sealed. Whether the air bubbles are removed by such a T-branch pipe is ascertained. For the T-branch pipe, the inside of the channels leading to the cell capturing device 1 was previously filled with degassed pure water. When pure water mixed with air flowed as a processing liquid, the air bubbles mixed in the processing liquid was discharged to the processing liquid channel 32 without separating the air bubbles in the upward pipe part at the branched part in the T-branch pipe. As a result, the processing liquid mixed with air was supplied to the cell capturing device 1.

### Reference Signs List

1... Cell Capturing Device, 3, 4, 9, 31, 32... Channel, 5... Processing Liquid Storage Container, 10... Blood Storage Container (Test Liquid Storage Container), 20A to 20H... Pre-processing Unit, 23... Foreign Matter Removal Filter, 57... Filter, 100... Cell Capturing Apparatus

## Claims

1. A cell capturing apparatus comprising:
a test liquid storage container configured to store a test liquid containing cells;
a cell capturing device including an introduction channel configured to introduce the test liquid or a processing liquid for processing the cells in the test liquid into an inside, a discharge channel configured to discharge the test liquid or the processing liquid to an outside, and a filter provided with a plurality of through-holes in a thickness direction, the filter being disposed on a channel between the introduction channel and the discharge channel so that the test liquid or the processing liquid passes through the through-holes;
a test liquid channel configured to connect the test liquid storage container and the introduction channel of the cell capturing device;
a processing liquid storage container configured to store the processing liquid for processing cells captured on the filter by passing through the filter;
a processing liquid channel configured to connect the processing liquid storage container and the introduction channel of the cell capturing device;
means for selection configured to select a liquid supplied to the cell capturing device from the test liquid and the processing liquid;
means for liquid supply configured to supply the test liquid from the test liquid storage container or the processing liquid from the processing liquid storage container to the cell capturing apparatus based on a selection result of the means for selection; and
a pre-processing unit including an air bubble accumulated region formed therein and disposed on the processing liquid channel and/or on the test liquid channel, the air bubble accumulated region having a larger diameter than a diameter of the provided channel, and including a foreign matter removal filter provided with a plurality of through-holes in a thickness direction, the foreign matter removal filter being disposed in the air bubble accumulated region so that the processing liquid or the test liquid passes through the through-holes.

2. The cell capturing apparatus according to claim 1, wherein an introduction port of the processing liquid or of the test liquid to the air bubble accumulated region is disposed on an upper side above a discharge port of the air bubble accumulated region in a height direction.

3. The cell capturing apparatus according to claim 1 or 2, wherein a main component of the foreign matter removal filter is a metal.

4. The cell capturing apparatus according to any one of claims 1 to 3, wherein, when the pre-processing unit is provided on the processing liquid channel, a cross-sectional area viewed in a moving direction of the processing liquid in the air bubble accumulated region is equal to or larger than a cross-sectional area viewed in a moving direction of the processing liquid in the introduction channel of the cell capturing device.

5. The cell capturing apparatus according to any one of claims 1 to 3, wherein, when the pre-processing unit is provided on the test liquid channel, a cross-sectional area viewed in a moving direction of the test liquid in the air bubble accumulated region is equal to or larger than a cross-sectional area viewed in a moving direction of the test liquid in the introduction channel of the cell capturing device.

6. The cell capturing apparatus according to any one of claims 1 to 5, wherein the pre-processing unit is configured by a member having a same shape as a shape of the cell capturing device except the filter.

7. The cell capturing apparatus according to any one of claims 1 to 6, wherein the pre-processing unit is provided on each of the processing liquid channel and the test liquid channel.

8. A cell capturing device provided with a pre-processing unit, the cell capturing device comprising:
one or more introduction channels configured to introduce a test liquid containing cells or a processing liquid for processing the cells in the test liquid into an inside;
a discharge channel configured to discharge the test liquid or the processing liquid to an outside;
a filter provided with a plurality of through-holes in a thickness direction and disposed on a channel between the introduction channel and the discharge channel so that the test liquid or the processing liquid passes through the through-holes; and
a pre-processing unit including an air bubble accumulated region formed therein and disposed on at least one introduction channel, the air bubble accumulated region having a larger diameter than a diameter of the introduction channel, and including a foreign matter removal filter provided with a plurality of through-holes in a thickness direction, the foreign matter removal filter being disposed in the air bubble accumulated region so that the processing liquid or the test liquid passes through the through-holes.

9. The cell capturing device provided with a pre-processing unit according to claim 8, wherein an introduction port of the test liquid or of the processing liquid to the air bubble accumulated region in the pre-processing unit is disposed on an upper side above a discharge port of the air bubble accumulated region in a height direction.

10. The cell capturing device provided with a pre-processing unit according to claim 8 or 9, wherein a main component of the foreign matter removal filter is a metal.

11. The cell capturing device provided with a pre-processing unit according to any one of claims 8 to 10, wherein a cross-sectional area viewed in a moving direction of the processing liquid in the air bubble accumulated region is equal to or larger than a cross-sectional area viewed in the moving direction of the processing liquid in the introduction channel.

12. The cell capturing device provided with a pre-processing unit according to any one of claims 8 to 11, wherein a cross-sectional area viewed in a moving direction of the test liquid in the air bubble accumulated region is equal to or larger than the cross-sectional area viewed in the moving direction of the test liquid in the introduction channel.

13. The cell capturing device provided with a pre-processing unit according to any one of claims 8 to 12, wherein
the plurality of introduction channels are provided, and
the pre-processing unit is provided on each introduction channel.

14. A pre-processing unit comprising:
an introduction channel configured to introduce a test liquid containing cells into an inside;
an air bubble accumulated region formed therein and disposed on the introduction channel, the air bubble accumulated region having a larger diameter than a diameter of the introduction channel; and
a foreign matter removal filter provided with a plurality of through-holes in a thickness direction, the foreign matter removal filter being disposed in the air bubble accumulated region so that the test liquid passes through the through-holes, wherein
a hole diameter of the foreign matter removal filter is 15 µm to 100 µm.

15. The pre-processing unit according to claim 14, wherein an introduction port of the test liquid to the air bubble accumulated region is disposed on an upper side above a discharge port of the air bubble accumulated region in a height direction.

16. A pre-processing unit comprising:
one or more introduction channels configured to introduce a processing liquid for processing cells in a test liquid into an inside;
an air bubble accumulated region formed therein and disposed on each introduction channel, the air bubble accumulated region having a larger diameter than a diameter of the introduction channel; and
a foreign matter removal filter provided with a plurality of through-holes in a thickness direction, the foreign matter removal filter being disposed in the air bubble accumulated region so that the test liquid passes through the through-holes, wherein
a hole diameter of the foreign matter removal filter is 1 µm to 5 µm.

17. The pre-processing unit according to claim 16, wherein an introduction port of the test liquid to the air bubble accumulated region is disposed on an upper side above a discharge port of the air bubble accumulated region in a height direction.

18. The pre-processing unit according to any one of claims 14 to 17, wherein a main component of the foreign matter removal filter is a metal.

19. The pre-processing unit according to claim 14 or 15, wherein a cross-sectional area viewed in a moving direction of the test liquid in the air bubble accumulated region is equal to or larger than a cross-sectional area viewed in a moving direction of the test liquid in the introduction channel.

20. The pre-processing unit according to claim 16 or 17, wherein a cross-sectional area viewed in a moving direction of the processing liquid in the air bubble accumulated region is equal to or larger than a cross-sectional area viewed in a moving direction of the processing liquid in the introduction channel.
